# EUROPÄISCHE PATENTSCHRIFT

(11) **EP 1 044 671 B1**
(45) Veröffentlichungstag und Bekanntmachung des Hinweises auf die Patenterteilung: **16.11.2005**
(21) Anmeldenummer: 00106795.8
(22) Anmeldetag: 30.03.2000
(51) Int. Cl.: A61K 6/02, A61K 7/16, A61L 27/02

(54) **Verwendung einer Suspension zur Behandlung von Hartgewebe und Hartgewebe-Ersatzmaterialien**
Use of a suspension for treatment of hard tissue and of prosthetic materials
Utilisation d'une Suspension pour le traitement de tissu dur et des matériaux de prothèse

(30) Priorität: 11.04.1999 DE 19916155
(43) Veröffentlichungstag der Anmeldung: 18.10.2000
(73) Patentinhaber: OROCHEMIE, Dürr + Pflug GmbH & Co. KG, 70806 Kornwestheim (DE); Dürr Dental GmbH & Co. KG, 74321 Bietigheim-Bissingen (DE); Hahn, Rainer, Dr., 72074 Tübingen (DE)
(72) Erfinder: Hahn, Rainer, 72074 Tübingen (DE); Heermann, Dieter, 71282 Hemmingen (DE); Brauner, Peter, 71563 Affalterbach (DE)
(74) Vertreter: Ostertag, Reinhard

(56) Entgegenhaltungen:
- EP-A- 0 414 128
- EP-A- 0 431 672
- DE-A- 4 406 323
- US-A- 4 331 422
- US-A- 5 124 143
- US-A- 5 328 682
- ZA-A- 8 308 422
- DATABASE WPI Derwent Publications Ltd., London, GB; AN 1987252489 XP002159113 & JP 62 172099 A (DAICEL CHEM IND), 29. Juli 1987 (1987-07-29)
- DATABASE WPI Week 197722, Derwent Publications Ltd., London, GB; Class A11, AN 1977-38593Y & JP 50 104 208 A (LION FAT & OIL CO LTD) 18 August 1975

## Beschreibung

Die Erfindung betrifft die Verwendung einer wässrigen Suspension zur Behandlung natürlichen Hartgewebes, wie Dentin, Zahnschmelz, Zahnstein, Zahnzement sowie von Hartgewebe-Ersatzmaterialien wie Zahn- oder Knochenersatzmaterialien oder Implantaten, mit in Wasser oder einer wässrigen Lösung suspendierten abrasiven Hartpartikeln im Zusammenhang mit der Anwendung oszillierender Geräte oder Instrumente, insbesondere im Ultraschallbereich.

In jüngster Zeit gewinnt die Ultraschallpräparation natürlicher Hartgewebe zunehmend an Bedeutung. Bei dieser kommen Werkzeuge zum Einsatz, die mit Hilfe eines Ultraschall-Schwingers in Oszillation versetzt werden und die über eine Suspension der eingangs genannten Art an das zu behandelnde Hartgewebe angekoppelt sind. Ein Beispiel hierfür ist in der DE-OS 44 06 323 beschrieben. Die dort erwähnte Suspension war insofern nicht stabil, als die Hartpartikel sich im Laufe der Zeit absetzen konnten, so daß die Suspension in einem auswechselbaren Vorratsbehälter ständig gerührt oder durch Gasdurchflutung in Schwebe gehalten werden mußten. Dies ist sehr aufwendig und führt auch nicht immer zu dem gewünschten Ergebnis.

In der EP-A-431 672 ist eine Zahnpasta beschrieben, die eine wässrige Dispersion von Hartpartikeln in einer Grundmasse aufweist, welche Wasser, Glyzerin, organische Dispergiermittel und Tonerde umfaßt. Diese Grundmasse hält die Hartpartikel in Suspension, wenn sie einmal in der Grundmasse verteilt sind.

In der US-A-5 124 143 ist eine Zahnpaste beschrieben, welche eine Grundmasse aus Glyzerin und Wasser umfaßt. In dieser befindet sich synthetisch hergestellte ausgefällte Kieselsäure, welche die Viskosität der Grundmasse erhöht und nicht zu starke abrasive Eigenschaften aufweist. Zusätzlich sind Hartpartikel in Form von Perlit enthalten.

In der EP-A-0 414 128 ist eine belaghemmende Zahnpaste beschrieben, welche als Poliermittel Aluminiumoxid-Trihydrat enthält. Die Größe dieser Hartpartikel ist überwiegend kleiner als 50 µm mit einem Mittelwert von etwa 1-10 µm. Zur Einstellung der Konsistenz der Zahnpaste sind nichtionische Polysaccharidderivate vorgesehen. Mehrwertige Alkohole dienen als Feuchthaltemittel.

In der JP-A-6217 2099 ist ein abrasives Reinigungsmaterial offenbart, dessen Grundmasse Wasser, Zellulose, Kieselsäure und Alkohol umfaßt. In dieser Grundmasse sind Hartpartikel verteilt, welche SiO₂-Verbindungen, Karbide oder Oxide umfassen.

In der US-A-5 328 682 ist ein abrasives Mundwasser beschrieben, welches eine Grundmasse aus Wasser und einem Dispergiermittel wie Ton, Polysaccarid oder Zellulose sowie mehrwertige Alkohole als Feuchthaltemittel enthält. Als Material für die abrasiven Partikel werden Karbonate, Phosphate, Oxide, Kunstharze und Mischungen dieser Materialien vorgeschlagen.

Die JP-A-501 042 08 betrifft ein flüssiges Reinigungsmittel mit abrasiven Tonpartikeln. Das Reinigungsmittel umfaßt ferner ein Dispergiermittel, z.B. Xanthan oder eine Zellulose sowie Äthylenglykol.

Aufgabe der vorliegenden Erfindung ist es, eine Suspension der eingangs genannten Art so auszugestalten, daß sie werkseitig herstell- und abfüllbar, dauerhaft lagerfähig und versendbar ist, ohne daß die Gefahr des Absetzens der Hartpartikel besteht, und die trotzdem mit geringem Aufwand pumpfähig ist.

Diese Aufgabe wird erfindungsgemäß gelöst, durch eine Verwendung mit den im Anspruch 1 angegeben Merkmalen.

Erfindungsgemäß läßt sich eine wässrige Suspension erhalten, die in der gewünschten Weise dauerhaft lagerfähig bleibt und sich nicht entmischt. Sie stellt ein Konzentrat dar, welches problemlos am Ort der Behandlung selbst, also mit Hilfe des Behandlungsgerätes oder in der Mundhöhle des Patienten im Handstück vor Austritt in die Mundhöhle mit Wasser in der entsprechenden Menge verdünnt wird.

Die nachfolgend näher erläuterten vorteilhaften Ausführungsbeispiele sind in drei Gruppen unterteilt: Die erste Konzentrat-Suspensiongruppe wird vorzugsweise zur Oberflächenglättung oder zur Entfernung von Auflagerungen auf Hartgewebsoberflächen oder zur Politur eingesetzt. Die zweite Gruppe eignet sich besonders für die abtragende Bearbeitung bzw. Präparation von Hartgewebsoberflächen oder von Zahn- oder Knochenersatzwerkstoffen. Die dritte Gruppe schließlich wird im wesentlichen zur Bearbeitung von Implantatoberflächen im Rahmen der Entfernung von harten oder weichen Belägen eingesetzt.

### Zunächst zur ersten Gruppe:

Hier empfiehlt es sich, wenn die Konzentrat-Suspension zwischen 5 und 20 Gewichtsprozent, vorzugsweise zwischen 8 und 12 Gewichtsprozent, Hartpartikel enthält. Als Hartpartikel kommen insbesondere in Frage Kalziumphosphate, insbesondere Apatite, hierbei wiederum bevorzugt Fluorapatit oder Hydroxylapatit, Silikatkeramiken oder Gläser, Oxidkeramik, z.B. Aluminiumoxidkeramikpartikel. Die Korngröße der Hartpartikel beträgt im Median bis maximal 100 bis 150 µm, vorzugsweise ca. 25 µm und nochmals vorzugsweise etwa 5 bis 10µm.

Für die zweite Gruppe von Konzentrat-Suspensionen haben sich folgende Charakteristiken besonders bewährt:

Sie enthalten zwischen 5 und 45 Gewichtsprozent, vorzugsweise zwischen 20 und 35 Gewichtsprozent, Hartpartikel. Als Materialien kommen bevorzugt in Frage Oxidkeramiken, z.B. Zirkonoxidkeramikpartikel, Nichtoxidkeramiken, z.B. Borcarbid, Bornitrid, Siliziumnitrid. Aufgrund der vorteilhaften grünen, nicht schwarzen Farbe eignet sich ganz besonders Siliziumkarbid. Die mittlere Teilchengröße der Hartpartikel bei dieser zweiten Gruppe liegt vorzugsweise zwischen etwa 5 µm und 100 µm, vorzugsweise etwa 25 µm und etwa 100 µm, nochmals vorzugsweise zwischen 40 µm und 50 µm.

Für die dritte Gruppe von Konzentrat-Suspensionen empfehlen sich Hartpartikel aus Kunststoff, z.B. Polysulfonpartikel, bzw. Kunststoffprecursoren, wie z.B. Polysiloxane oder Polysilazane, sowie pyrolytisch hergestellte Keramiken. Es eigenen sich für diese Gruppe aber auch Hartpartikel, wie sie oben für die Gruppe 1 genannt wurden, oder - falls ein Materialabtrag erwünscht ist - Hartpartikel, wie sie oben für die Gruppe 2 genannt wurden.

Als Dispergier- und Verdickungsmittel kommen unterschiedliche Substanzen in Frage. Beispielsweise kann die Suspension zwischen 2 und 10 Gewichtsprozent, vorzugsweise zwischen 3 und 8 Gewichtsprozent, hochdisperse Kieselsäure enthalten. Gute Resultate ergeben sich auch dann, wenn als Dispergier- und Verdickungsmittel zwischen 0,1 und 4 Gewichtsprozent, vorzugsweise zwischen 0,3 und 2,5 Gewichtsprozent Bentonit verwendet werden.

Bevorzugt als Dispergier- und Verdickungsmittel werden zwischen 0,15 und 3, stärker bevorzugt zwischen 0,25 und 1,5, Gewichtsprozent Polysaccharid, bei dem es sich zweckmäßigerweise um Xanthan handelt.

Zur Aufrechterhaltung der Keimfreiheit der Konzentrat-Suspension auch über längere Lagerzeiten hinweg kann sie geeignete Konservierungsmittel enthalten.

Hierzu gehören PHB-Ester (Parabene) wie Methyl-, Ethyl-, Propyl- oder Butylester der Parahydroxybenzoesäure, Alkohole wie Ethanol, Propanole oder Phenoxyethanol oder Mischungen hiervon.

An und für sich wäre es besonders günstig, wenn der pH-Wert der Konzentrat-Suspension bei etwa 11,0 liegt. Bei diesem pH-Wert erreichen die Hartpartikel, wie Versuche gezeigt haben, ein negatives Potentional von etwa -25 mV. Durch Abstoßung der gleichnamigen Ladungen auf den Partikeloberflächen ergibt sich eine sehr große Stabilisierungswirkung für die Konzentrat-Suspension. So ist es möglich, eine stabile Konzentrat-Suspension auch im pH-Bereich zwischen 5 und 9, bevorzugt zwischen 6 und 8, zu formulieren. Eine solche Konzentrat-Suspension kann direkt in der Mundhöhle zur Anwendung gebracht werden.

Beispiele werden nachstehend näher beschrieben.

### Beispiel 1

Eine Konzentrat-Suspension mit besonders stark abrasiver Wirkung weist folgende Zusammensetzung auf:

| Chemische Bezeichnung | Gehalt (Gewichtsprozent) |
|---|---|
| Siliziumkarbid | 25 |
| Xanthan | 0,55 |
| PHB-Ester-Gemisch | 0,15 |
| Glyzerin | 5 |
| gereinigtes Wasser | ad 100 |

### Beispiel 2

Eine weitere Konzentrat-Suspension mit besonders stark abrasiver Wirkung weist folgende Zusammensetzung auf:

| Chemische Bezeichnung | Gehalt (Gewichtsprozent) |
|---|---|
| Siliziumkarbid | 30 |
| Xanthan | 0,5 |
| PHB-Ester-Gemisch | 0,2 |
| Glyzerin | 12 |
| Phenoxyethanol | 0,5 |
| Ethanol | 3 |
| gereinigtes Wasser | ad 100 |

Bei den Beispielen 1 und 2 erhält man eine stark abrasive Wirkung, wenn man den Durchmesser der Siliziumkarbid-Partikel im Bereich von etwa 40 bis 50 µm wählt. Teilchendurchmesser von etwa 5 bis 10 µm führen zu einem finierenden vorsichtigen Abtrag.

### Beispiel 3

Eine weniger stark abrasive, insbesondere für Polierzwecke geeignete Konzentrat-Suspension weist die folgende Zusammensetzung auf:

| Chemische Bezeichnung | Gehalt (Gewichtsprozent) |
|---|---|
| Hydroxylapatit | 8 |
| Xanthan | 0,55 |
| Glyzerin | 15 |
| PHB-Ester-Gemisch | 0,15 |
| gereinigtes Wasser | ad 100 |

### Beispiel 4

Eine weitere weniger stark abrasive, insbesondere für Polierzwecke geeignete Konzentrat-Suspension weist die folgende Zusammensetzung auf:

| Chemische Bezeichnung | Gehalt (Gewichtsprozent) |
|---|---|
| Hydroxylapatit | 8 |
| Xanthan | 0,6 |
| Glyzerin | 12 |
| PHB-Ester-Gemisch | 0,2 |
| Phenoxyethanol | 0,5 |
| Ethanol | 3 |
| gereinigtes Wasser | ad 100 |

In den Zusammensetzungen nach den Beispielen 3 und 4 kann das Hydroxylapatit als Hartstoff auch durch Fluorapatit ersetzt werden. Wählt man die Teilchengrösse in der Nachbarschaft von 50 µm erhält man ein forciertes Polieren, bei Teilchengrössen im Bereich von etwa 5 bis 10 µm ein Polieren.

### Beispiel 5

Eine weitere weniger stark abrasive, insbesondere für ein Polieren von Implantaten geeignete Konzentrat-Suspension weist die folgende Zusammensetzung auf:

| Chemische Bezeichnung | Gehalt (Gewichtsprozent) |
|---|---|
| Hydroxylapatit | 2,5 |
| Polysiloxan | 5,5 |
| Xanthan | 0,6 |
| Glyzerin | 12 |
| PHB-Ester-Gemisch | 0,2 |
| Phenoxyethanol | 0,5 |
| Ethanol | 3 |
| gereinigtes Wasser | ad 100 |

## Patentansprüche

1. Verwendung einer wässrigen Suspension erhalten durch
Verdünnen einer Konzentrat-Suspension mit Wasser an Behandlungs ist selbst, welche bezogen auf die Gesamtmasse der Konzentrat-Suspension enthält:
a) zwischen 5 und 45 Gewichtsprozent Hartpartikel,
b) zwischen 2 und 40 Gewichtsprozent einer Alkoholkomponente, welche mindestens einen ein- oder mehrwertigen Alkohol oder ein Gemisch ein- oder mehrwertiger Alkohole umfaßt, und
c) zwischen 0,1 und 10 Gewichtsprozent Dispergier- und Verdickungsmittel,
als Kopplungsmittel bei der Anwendung oszillierender Geräte oder Instrumente insbesondere im Ultraschallbereich für die Behandlung natürlichen Hartgewebes, wie Dentin, Zahnschmelz, Zahnstein, Zahnzement sowie von Hartgewebe-Ersatzmaterialien wie Zahn- oder Knochenersatzmateralien oder Implantaten.

2. Verwendung nach Anspruch 1, **dadurch gekennzeichnet,**
**daß** die Konzentrat-Suspension zwischen 5 und 20 Gewichtsprozent Hartpartikel enthält.

3. Verwendung nach Anspruch 2, **dadurch gekennzeichnet,**
**daß** die Konzentrat-Suspension zwischen 8 und 12 Gewichtsprozent Hartpartikel enthält.

4. Verwendung nach einem der Ansprüche 1 bis 3, **dadurch**
**gekennzeichnet, daß** die Konzentrat-Suspension als Hartpartikel Kalziumphosphate, insbesondere Apatite, bevorzugt Fluorapatit oder Hydroxylapatit, Silikatkeramiken, Gläser, Oxidkeramik, z.B. Aluminiumoxidkeramik, enthält.

5. Verwendung nach einem der Ansprüche 1 bis 4,
**dadurch gekennzeichnet, daß** die mittlere maximale Teilchengröße der Hartpartikel 100 bis 150µm, bevorzugt ca. 25 µm, nochmals bevorzugt 5 bis 10 µm, beträgt.

6. Verwendung nach Anspruch 1, **dadurch gekennzeichnet,**
**daß** die Konzentrat-Suspension zwischen 20 und 35 Gewichtsprozent Hartpartikel enthält.

7. Verwendung nach Anspruch 6, **dadurch gekennzeichnet,**
**daß** die Konzentrat-Suspension als Hartpartikel Oxidkeramiken, z.B. Zirkonoxidkeramik, Nichtoxidkeramiken, z.B. Borkarbid, Bornitrid, Siliziumnitrid, enthält.

8. Verwendung nach Anspruch 6, **dadurch gekennzeichnet,**
**daß** die Konzentrat-Suspension als Hartpartikel Siliziumkarbid enthält.

9. Verwendung nach einem der Ansprüche 6 bis 8, **dadurch**
**gekennzeichnet, daß** die mittlere Teilchengröße der Hartpartikel zwischen 5 µm und ca. 100 µm, vorzugsweise zwischen 25 µm bis ca. 100 µm, nochmals vorzugsweise zwischen 40 µm und 50 µm, liegt.

10. Verwendung nach einem der Ansprüche 1 bis 9, **dadurch**
**gekennzeichnet, daß** unter den Hartpartikeln Partikel aus Kunststoff, z.B. Polysulfon-Partikel, oder aus Kunststoffprecursoren, z.B. Polysiloxanen oder Polysilazanen oder pyrolytisch hergestellte Keramiken, sind oder die Hartpartikel ganz durch derartige Partikel gebildet sind.

11. Verwendung nach einem der vorhergehenden Ansprüche,
**dadurch gekennzeichnet, daß** die Konzentrat-Suspension als Dispergier- und Verdickungsmittel zwischen 2 und 10 Gewichtsprozent hochdisperse Kieselsäure enthält.

12. Verwendung nach Anspruch 11, **dadurch gekennzeichnet,**
**daß** die Konzentrat-Suspension zwischen 3 und 8 Gewichtsprozent hochdisperse Kieselsäure enthält.

13. Verwendung nach einem der vorhergehenden Ansprüche,
**dadurch gekennzeichnet, daß** die Konzentrat-Suspension als Dispergier- und Verdickungsmittel zwischen 0,1 und 4 Gewichtsprozent Bentonit enthält.

14. Verwendung nach Anspruch 13, **dadurch gekennzeichnet,**
**daß** die Konzentrat-Suspension zwischen 0,3 und 2,5 Gewichtsprozent Bentonit enthält.

15. Verwendung nach einem der vorhergehenden Ansprüche,
**dadurch gekennzeichnet, daß** die Konzentrat-Suspension als Dispergier- und Verdickungsmittel zwischen 0,15 und 3 Gewichtsprozent Polysaccharide enthält.

16. Verwendung nach Anspruch 15, **dadurch gekennzeichnet,**
**daß** die Konzentrat-Suspension zwischen 0,25 und 1,5 Gewichtsprozent Polysaccharide enthält.

17. Verwendung nach Anspruch 15 oder 16, **dadurch gekenn**
**zeichnet, daß** die Konzentrat-Suspension als Polysaccharid Xanthan enthält.

18. Verwendung nach einem der vorhergehenden Ansprüche,
**dadurch gekennzeichnet, daß** die Konzentrat-Suspension mindestens ein Konservierungsmittel enthält.

19. Verwendung nach einem der vorhergehenden Ansprüche
**dadurch gekennzeichnet, daß** ihr pH-Wert zwischen 5 und 9, bevorzugt zwischen 6 und 8, liegt.

20. Verwendung.nach einem der vorhergehenden Ansprüche
**dadurch gekennzeichnet, daß** die Alkoholkomponente mindestens einen Alkohol aus der nachstehenden Gruppe enthält: Ethanol, Propanole, Glycerin, Propylenglykole, Diethylenglykol.

21. Verwendung nach einem der vorhergehenden Ansprüche,
**dadurch gekennzeichnet, daß** die Konzentrat-Suspension eine Mischung aus anorganischen Hartpartikeln nach Anspruch 6 oder 7 und organischen Hartpartikeln nach Anspruch 10 umfaßt.

## Claims

1. Use of an aqueous suspension obtained by diluting a suspension concentrate with water at the site of the treatment itself, which contains the following, based on the total weight of the suspension concentrate:
a) between 5 and 45 wt.% of hard particles,
b) between 2 and 40 wt.% of an alcohol component comprising at least one monohydric or polyhydric alcohol or a mixture of monohydric or polyhydric alcohols, and
c) between 0.1 and 10 wt.% of dispersants and thickeners,
as a coupling agent in the application of oscillating devices or instruments, especially in the ultrasound region, for the treatment of natural hard tissue such as dentine, dental enamel, dental calculus or dental cement, and hard tissue prostheses such as dental or bone prostheses or implants.

2. Use according to Claim 1, **characterized in that** the suspension concentrate contains between 5 and 20 wt.% of hard particles.

3. Use according to Claim 2, **characterized in that** the suspension concentrate contains between 8 and 12 wt.% of hard particles.

4. Use according to one of Claims 1 to 3, **characterized in that** the suspension concentrate contains, as hard particles, calcium phosphates, especially apatites and preferably fluorapatite or hydroxyapatite, silicate ceramics, glasses or oxide ceramic, e.g. aluminium oxide ceramic.

5. Use according to one of Claims 1 to 4, **characterized in that** the maximum mean size of the hard particles is 100 to 150 µm, preferably approx. 25 µm and particularly preferably 5 to 10 µm.

6. Use according to Claim 1, **characterized in that** the suspension concentrate contains between 20 and 35 wt.% of hard particles.

7. Use according to Claim 6, **characterized in that** the suspension concentrate contains, as hard particles, oxide ceramics, e.g. zirconium oxide ceramic, or non-oxide ceramics, e.g. boron carbide, boron nitride or silicon nitride.

8. Use according to Claim 6, **characterized in that** the suspension concentrate contains silicon carbide as hard particles.

9. Use according to one of Claims 6 to 8, **characterized in that** the mean size of the hard particles is between 5 µm and approx. 100 µm, preferably between 25 µm and approx. 100 µm and particularly preferably between 40 µm and 50 µm.

10. Use according to one of Claims 1 to 9, **characterized in that** the hard particles include particles of plastic, e.g. polysulfone particles, or plastic precursors, e.g. polysiloxanes or polysilazanes, or ceramics prepared by pyrolysis, or consist entirely of such particles.

11. Use according to one of the preceding claims, **characterized in that** the suspension concentrate contains, as dispersants and thickeners, between 2 and 10 wt.% of highly disperse silicic acid.

12. Use according to Claim 11, **characterized in that** the suspension concentrate contains between 3 and 8 wt.% of highly disperse silicic acid.

13. Use according to one of the preceding claims, **characterized in that** the suspension concentrate contains, as dispersants and thickeners, between 0.1 and 4 wt.% of bentonite.

14. Use according to Claim 13, **characterized in that** the suspension concentrate contains between 0.3 and 2.5 wt.% of bentonite.

15. Use according to one of the preceding claims, **characterized in that** the suspension concentrate contains, as dispersants and thickeners, between 0.15 and 3 wt.% of polysaccharides.

16. Use according to Claim 15, **characterized in that** the suspension concentrate contains between 0.25 and 1.5 wt.% of polysaccharides.

17. Use according to Claim 15 or 16, **characterized in that** the suspension concentrate contains xanthan as the polysaccharide.

18. Use according to one of the preceding claims, **characterized in that** the suspension concentrate contains at least one preservative.

19. Use according to one of the preceding claims, **characterized in that** the pH of the suspension concentrate is between 5 and 9, preferably between 6 and 8.

20. Use according to one of the preceding claims, **characterized in that** the alcohol component contains at least one alcohol from the following group: ethanol, propanols, glycerol, propylene glycols and diethylene glycol.

21. Use according to one of the preceding claims, **characterized in that** the suspension concentrate comprises a mixture of inorganic hard particles according to Claim 6 or 7 and organic hard particles according to Claim 10.

## Revendications

1. Utilisation d'une suspension aqueuse obtenue par dilution d'une suspension de concentré avec de l'eau sur le lieu d'utilisation lui-même, laquelle contient, sur la base de la masse totale de la suspension de concentré :
a) 5 à 45 pour-cent en masse de particules dures,
b) 2 à 40 pour-cent en masse d'un composant d'alcool ou composante alcoolique qui comporte au moins un alcool monovalent ou polyvalent ou un mélange d'alcools monovalents ou polyvalents, et
c) 0,1 à 10 pour-cent en masse d'agent dispersant et d'agent épaississant,
en tant qu'agent de couplage lors de l'utilisation d'appareils ou d'instruments à oscillation, en particulier dans le domaine des ultrasons, pour le traitement d'un tissu dur naturel, comme la dentine, l'émail, le tartre dentaire, le cément dentaire, ainsi que de matériaux de remplacement des tissus durs, comme les matériaux de prothèse dentaire ou osseuse, ou les implants.

2. Utilisation selon la revendication 1, **caractérisée en ce que** la suspension de concentré contient 5 à 20 pour-cent en masse de particules dures.

3. Utilisation selon la revendication 2, **caractérisée en ce que** la suspension de concentré contient 8 à 12 pour-cent en masse de particules dures.

4. Utilisation selon l'une des revendications 1 à 3, **caractérisée en ce que** la suspension de concentré contient en tant que particules dures, des phosphates de potassium, en particulier les apatites, de préférence la fluorapatite ou l'hydroxylapatite, des céramiques silicatées ou des verres, de la céramique oxyde, par exemple des particules de céramique d'oxyde d'aluminium.

5. Utilisation selon l'une des revendications 1 à 4, **caractérisée en ce que** la taille moyenne maximale des particules dures est comprise entre 100 et 150 µm, est de préférence d'environ 25 µm, de façon encore plus préférée comprise entre 5 et 10 µm.

6. Utilisation selon la revendication 1, **caractérisée en ce que** la suspension de concentré contient 20 à 35 pour-cent en masse de particules dures.

7. Utilisation selon la revendication 6, **caractérisée en ce que** la suspension de concentré contient, en tant que particules dures, des céramiques d'oxyde, par exemple de la céramique à l'oxyde de zirconium, des céramiques non oxydes, par exemple du carbure de bore, du nitrure de bore, du nitrure de silicium.

8. Utilisation selon la revendication 6, **caractérisée en ce que** la suspension de concentré contient, en tant que particules dures, du carbure de silicium.

9. Utilisation selon l'une des revendications 6 à 8, **caractérisée en ce que** la taille moyenne des particules dures est comprise entre 5 µm et environ 100 µm, de préférence entre 25 µm et environ 100 µm, de façon encore plus préférée entre 40 µm et 50 µm.

10. Utilisation selon l'une des revendications 1 à 9, **caractérisée en ce que** parmi les particules dures se trouvent des particules en matière synthétique, par exemple des particules de polysulfone, ou des précurseurs de matières synthétiques, par exemple des polysiloxanes ou des polysilazanes ou des céramiques fabriquées par procédé pyrolytique, ou **en ce que** les particules dures sont formées entièrement par des particules de ce type.

11. Utilisation selon l'une des revendications précédentes, **caractérisée en ce que** la suspension de concentré contient en tant qu'agent dispersant et qu'agent épaississant 2 à 10 pour-cent en masse d'acide silicique hautement dispersé.

12. Utilisation selon la revendication 11, **caractérisée en ce que** la suspension de concentré contient 3 à 8 pour-cent en masse d'acide silicique hautement dispersé.

13. Utilisation selon l'une des revendications précédentes, **caractérisée en ce que** la suspension de concentré contient en tant qu'agent dispersant et qu'agent épaississant 0,1 à 4 pour-cent en masse de bentonite.

14. Utilisation selon la revendication 13, **caractérisée en ce que** la suspension de concentré contient 0, 3 à 2,5 pour-cent en masse de bentonite.

15. Utilisation selon l'une des revendications précédentes, **caractérisée en ce que** la suspension de concentré contient en tant qu'agent dispersant et épaississant 0,15 à 3 pour-cent en masse de polysaccharides.

16. Utilisation selon la revendication 15, **caractérisée en ce que** la suspension de concentré contient 0,25 à 1,5 pour-cent en masse de polysaccharides.

17. Utilisation selon la revendication 15 ou 16, **caractérisée en ce que** la suspension de concentré contient, en tant que polysaccharide, du xanthane.

18. Utilisation selon l'une des revendications précédentes, **caractérisée en ce que** la suspension de concentré contient au moins un conservateur.

19. Utilisation selon l'une des revendications précédentes, **caractérisée en ce que** sa valeur de pH est comprise entre 5 et 9, de préférence entre 6 et 8.

20. Utilisation selon l'une quelconque des revendications précédentes, **caractérisée en ce que** le composant d'alcool contient au moins un alcool issu du groupe suivant : éthanol, propanol, glycérine, propylèneglycol, diéthylèneglycol.

21. Utilisation selon l'une des revendications précédentes, **caractérisée en ce que** la suspension de concentré contient un mélange de particules dures inorganiques selon la revendication 6 ou 7 et de particules dures organiques selon la revendication 10.
